(19) Europäisches Patentamt
European Patent Office
Office européen des brevets

(11) Veröffentlichungsnummer: **0 224 523 B1**

(12) **EUROPÄISCHE PATENTSCHRIFT**

(45) Veröffentlichungstag der Patentschrift: **01.03.89**

(51) Int. Cl.⁴: **C 07 C 141/00**, C 07 C 141/08, C 07 F 9/09, C 11 D 1/29, C 11 D 1/34

(21) Anmeldenummer: **86903352.2**

(22) Anmeldetag: **28.05.86**

(86) Internationale Anmeldenummer: **PCT/EP 86/00319**

(87) Internationale Veröffentlichungsnummer: **WO 86/07356 (18.12.86 Gazette 86/27)**

# (54) TEILESTER VON 2-BENZYLALKANOLEN-(1) UND 2-BENZYLALKANOL-1-POLYGLYKOLETHERN SOWIE VERFAHREN ZU IHRER HERSTELLUNG.

(30) Priorität: **10.06.85 DE 3520709**

(43) Veröffentlichungstag der Anmeldung: **10.06.87 Patentblatt 87/24**

(45) Bekanntmachung des Hinweises auf die Patenterteilung: **01.03.89 Patentblatt 89/9**

(84) Benannte Vertragsstaaten: **DE**

(56) Entgegenhaltungen: **keine**

(73) Patentinhaber: **Henkel Kommanditgesellschaft auf Aktien, Postfach 1100 Henkelstrasse 67, D-4000 Düsseldorf-Holthausen (DE)**

(72) Erfinder: **KRAUSE, Horst-Jürgen, Am Nettchesfeld 22, D-4000 Düsseldorf 13 (DE)**
Erfinder: **SYLDATK, Andreas, Am Nettchesfeld 25, D-4000 Düsseldorf 13 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

## Beschreibung

Gegenstand der Erfindung ist die neue Verbindungsklasse der Schwefelsäureteilester und Phosphorsäureteilester und ihrer Salze von 2-Benzylalkanolen und 2-Benzylalkanol-1-polyglykolethern, ein Verfahren zu ihrer Herstellung sowie ihre Verwendung als grenzflächenaktive Verbindungen.

2-Benzylalkanole-(1) sind seit längerem bekannt. Sie wurden von P. MASTAGLI, «Comptes Rendus», *204* (1937), Seiten 1656-1658 durch gemischte Aldolkondensation von Aldehyden mit Benzylalkohol und anschliessende Hydrierung erhalten. C. WEIZMANN, «Chemistry and Industry», 1937, Seiten 587-591 und E. F. PRATT, «Journal of the American Chemical Society», *76* (1954), Seiten 52-56 beschreiben die Herstellung von 2-Benzylalkanolen aus Benzylalkohol und Alkoholen mit maximal 7 C-Atomen mittels GUERBET-Reaktion.

Die Herstellung entsprechender GUERBET-Alkohole aus Alkanolen mit mehr als 7 C-Atomen auf diesem Wege ist noch nicht beschrieben. Nicht bekannt sind ferner die Teilester der Schwefelsäure und Phosphorsäure von 2-Benzylalkanolen-(1) sowie von 2-Benzylalkanol-1-polyglykolethern. Es wurde nun gefunden, dass sie bzw. ihre wasserlöslichen Salze wertvolle Verbindungen mit grenzflächenaktiven Eigenschaften darstellen.

Gegenstand der Erfindung sind demnach Teilester von Benzylalkanolen und 2-Benzylalkanol-1-polyglykolethern der allgemeinen Formel I

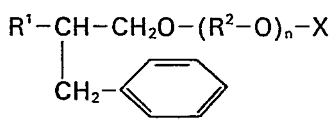 (I)

in der $R^1$ einen Alkylrest mit 6 bis 22 C-Atomen, $R^2$ einen Alkylenrest mit 2-4 C-Atomen, n ganze Zahlen von 0-10, X den Rest $SO_3Y$ oder $PO_3Y_2$ und Y Wasserstoff, ein Alkalimetall, Erdalkalimetall, Ammonium oder eine organische Ammoniumverbindung bedeuten.

$R^1$ steht vorzugsweise für einen Alkylrest mit 8-18 C-Atomen. $R^2$ steht vorzugsweise für eine Ethylengruppe oder Propylengruppe, d.h. der Ausdruck ($R^2$-O) steht für eine Ethylenglykolethergruppe (EO) und/oder eine Propylenglykolethergruppe (PO). In einer Verbindung der Formel I können daher nur EO-Gruppen oder nur PO-Gruppen oder auch beide Gruppen in beliebiger Reihenfolge stehen. Die Zahl n beträgt vorzugsweise 1-5. Als salzbindende Kationen kommen insbesondere Na, K, $NH_4$ und Mg in Frage, ferner Mono-, Di- oder Trialkanolamine von $C_2$-$C_3$-Alkanolen.

Beispiele für derartige Verbindungen sind:

2-Benzyloctanol-$SO_3Y$
2-Benzyldecanol-$SO_3Y$
2-Benzyldodecanol-$SO_3Y$
2-Benzyltetradecanol-$SO_3Y$
2-Benzylhexadecanol-$SO_3Y$
2-Benzyloctadecanol-$SO_3Y$
2-Benzyloctanol-$(EO)_n$-$SO_3Y$
2-Benzyldecanol-$(EO)_n$-$SO_3Y$
2-Benzyldodecanol-$(EO)_n$-$SO_3Y$
2-Benzyltetradecanol-$(EO)_n$-$SO_3Y$
2-Benzylhexadecanol-$(EO)_n$-$SO_3Y$
2-Benzyloctadecanol-$(EO)_n$-$SO_3Y$
2-Benzyldecanol-$(EO)_n$-$PO_3Y_2$
2-Benzyldodecanol-$(EO)_n$-$PO_3Y_2$
2-Benzyltetradecanol-$(EO)_n$-$PO_3Y_2$
2-Benzylhexadecanol-$(EO)_n$-$PO_3Y_2$
2-Benzyloctadecanol-$(EO)_n$-$PO_3Y_2$
2-Benzyloctanol-$PO_3Y_2$
2-Benzyldecanol-$PO_3Y_2$
2-Benzyldodecanol-$PO_3Y_2$
2-Benzyltetradecanol-$PO_3Y_2$

Die Herstellung der Verbindungen erfolgt in einer 1. Stufe durch Erhitzen eines Gemisches aus Alkanol, enthaltend 8 bis 24 C-Atome, vorzugsweise 8 bis 18 C-Atome und Benzylalkohol auf Temperaturen von 150 bis 280°C in Gegenwart einer alkalisch reagierenden Alkalimetallverbindung (gemischte GUERBET-Reaktion). Um eine konkurrierende homologe GUERBET-Reaktion des Alkanols zu unterdrücken, wird der Benzylalkohol zweckmässigerweise im Überschuss eingesetzt. Dieser Überschuss kann bis zu 10fach molar sein, vorzugsweise ist er 2fach bis 3fach molar. Als alkalisch reagierende Katalysatoren kommen die Hydroxide, Carbonate, Borate, Silikate und Phosphate des Kaliums oder Natriums in Betracht, ebenso solche Alkalimetallverbindungen, die mit dem entstehenden Reaktionswasser unter Bildung der Hydroxide reagieren, z.B. Alkoholate, Oxide, Amide und Hydride. Vorzugsweise wird Kaliumhydroxid in Anteilen von 0,1 bis 0,25 Mol.-%, bezogen auf eingesetztes Alkanol, verwendet.

Die Ausbeute lässt sich erheblich steigern, wenn zusätzlich Hydrierungs- bzw. Dehydrierungskatalysatoren in feinverteilter Form anwesend sind. Beispiele hierfür sind Metalle der Nebengruppen I und VIII des periodischen Systems der Elemente, wie Cu, Fe, Ni, Co, Pt und Pd sowie Mischkatalysatoren, ferner Salze des Fe, Zn, Co, Mn und Cr. Als besonders geeignet haben sich Cu, Ni und Platinmetalle sowie deren Legierungen erwiesen. Der Zusatz an diesen Dehydrierungskatalysatoren kann 0,1 bis 5 Gew.-%, vorzugsweise 0,2 bis 2 Gew.-%, bezogen auf das Reaktionsgemisch, betragen.

Die GUERBET-Reaktion findet bei Temperaturen zwischen 150 bis 280°C, vorzugsweise zwischen 180 bis 250°C statt. Bei Temperaturen oberhalb des Siedepunktes der Alkohole (Octanol 194°, Decanol 229°, Benzylalkohol 205°) wird in einem Druckbehälter gearbeitet. Das bei der Reaktion gebildete Wasser wird zweckmässigerweise aus dem Reaktionsgemisch entfernt, beispielsweise durch Abdestillieren, wobei der Alkohol als Schleppmittel dient. Das abdestillierte Azeotrop wird vor der Rückführung in das Reaktionsgemisch entwässert, wobei wegen des geringen Dichteunterschiedes zwischen Benzylalkohol und Wasser zweckmässigerweise wasserentziehende Mittel zugesetzt werden. Als wasserentziehende Mittel eignen sich z.B. kristallwasserbindende Salze sowie konzentrierte Lösungen dieser Salze, wie Natriumsulfat.

Nach Abschluss der Reaktion, die je nach angewendeten Reaktionsbedingungen 0,5 bis 5 Stunden benötigt, wird der Katalysator abgetrennt, was durch Dekantieren oder Abfiltrieren erfolgen kann. Noch gelöstes Alkali kann zuvor neutralisiert werden. Das Reaktionsgut enthält im allgemeinen neben nicht umgesetztem Ausgangsmaterial, insbesondere überschüssigem Benzylalkohol, auch aliphatische GUERBET-Alkohole der Formel II

$$R^1-CH_2-CH_2-\underset{\displaystyle R^1}{\underset{|}{CH}}-CH_2OH \qquad (II)$$

die durch Selbstkondensation der Alkanole untereinander entstehen. Die 2-Benzylalkanole-(1) werden aus diesem Gemisch durch fraktionierte Destillation isoliert. In den Fällen, in denen die anwesenden aliphatischen GUERBET-Alkohole nicht stören und in Form ihrer Teilester bzw. alkoxylierten Teilester mitverwendet werden können, genügt es, wenn der überschüssige Benzylalkohol und andere Nebenprodukte abgetrennt werden. Als Nebenprodukt fallen hauptsächlich Alkalimetallsalze von Säuren, insbesondere Kaliumbenzoat an, das durch Dehydrierung des Benzylalkohols entstanden ist. Diese im Reaktionsgut unlöslichen Salze lassen sich zusammen mit dem Katalysator leicht mechanisch bzw. durch Auswaschen mit Wasser abtrennen.

Das in 1. Stufe gebildete 2-Benzylalkanol-(1) wird, sofern die Herstellung der Polyglykolethersulfate bzw. Polyglykoletherphosphate angestrebt wird, in an sich bekannter Weise mit Alkylenoxiden zu den entsprechenden Polyglykolethern umgesetzt. Als Alkylenoxide kommen insbesondere Ethylenoxid und Propylenoxid, ferner Butylenoxid sowie deren Gemische in Frage. Die Alkylenoxide können ausserdem in unterschiedlicher Reihenfolge zum Einsatz kommen, d.h. die 2-Benzylalkanole werden zunächst mit Ethylenoxid und anschliessend mit Propylenoxid oder gegebenenfalls Butylenoxid umgesetzt. Auch die umgekehrte Reihenfolge ist möglich und führt ebenfalls zu gut brauchbaren Produkten. Bei Einsatz von Alkylenoxid-Gemischen ergibt sich eine statistische Verteilung der Glykolethergruppen. Bei Alkoxylierungsgraden unter 4, insbesondere 1 bis 2, können auch grössere Mengen an freien Benzylalkanolen bzw. rein aliphatischen GUERBET-Alkoholen anwesend sein.

Die Alkoxylierung erfolgt bei Temperaturen von 60 bis 240° C, vorzugsweise 80 bis 200° C in Gegenwart saurer oder alkalischer Katalysatoren, wobei zweckmässigerweise in einem Druckgefäss gearbeitet wird. Saure Katalysatoren sind beispielsweise LEWIS-Säuren, wie Bortrifluorid oder Aluminiumchlorid. Bevorzugt sind alkalische Katalysatoren, wie die Alkoholate, Hydroxide, Oxide, Carbonate, Amide und Hydride von Alkali- oder Erdalkalimetallen. Ihre Menge beträgt im allgemeinen 0,1 bis 0,8, vorzugsweise 0,2 bis 0,6 Mol pro Mol an eingesetztem Benzylalkanol. Das Alkylenoxid wird gasförmig unter intensivem Rühren in das Reaktionsgut eingeleitet. Nach beendeter Umsetzung kann der Katalysator durch Neutralisation inaktiviert und das gebildete Salz mechanisch abgetrennt werden.

Die Überführung der 2-Benzylalkanole-(1) und ihrer Glykolether sowie der gegebenenfalls darin noch enthaltenen aliphatischen GUERBET-Alkohole bzw. deren Glykolether in die Halbester erfolgt mit bekannten Sulfatierungs- bzw. Phosphatierungsmitteln. Geeignet sind Schwefelsäure, Chlorsulfonsäure, Amidosulfonsäure und Schwefeltrioxid, das gasförmig und mit Inertgasen verdünnt oder in Lösungsmitteln, wie flüssiges Schwefeldioxid oder Dioxan, eingesetzt werden kann. Eine schonende, zu besonders hellfarbigen Produkten führende Sulfatierung wird insbesondere mit Chlorsulfonsäure und Amidosulfonsäure erzielt. Geeignete Phosphatierungsmittel sind z.B. Phosphorsäure, Phosphoroxychlorid und Phosphorpentaoxid.

Das Sulfatierungsmittel kann equimolar, im geringfügigen Überschuss, z.B. bis 1,2-molar, oder auch im Unterschuss angewandt werden. Insbesondere dann, wenn als Ausgangsmaterial ethoxylierte 2-Benzylalkanole und ihre Gemische mit ethoxylierten aliphatischen GUERBET-Alkoholen eingesetzt werden, kann das Sulfatierungsmittel auch im Unterschuss angewendet werden. Die im Reaktionsgemisch verbleibenden, nicht sulfatierten Glykolether besitzen ihrerseits reinigende und emulgierende Eigenschaften und können die Eigenschaften der Schwefelsäureteilester bzw. deren Salze erheblich steigern. Sulfiergrade von 80% bis knapp 100% können auch bei Anwendung equimolarer Mengen an Sulfatierungsmittel erhalten werden, insbesondere dann, wenn die Sulfatierung unter schonenden Bedingungen durchgeführt wird. Auch in diesen Fällen stören die nichtsulfatierten Bestandteile in der Regel nicht, sondern verbessern vielfach die Gebrauchseigenschaften der Verfahrensprodukte.

Bei der Überführung in die Phosphorsäureteilester werden neben den entsprechenden Monoestern der Formel I Diester der Formel III

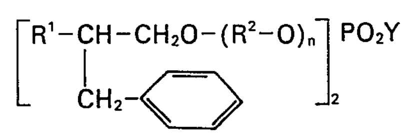

(III)

gebildet. In dieser Formel haben die Symbole die vorstehend angegebene Bedeutung. Der Anteil dieser Diester im Gemisch unterliegt statistischen Gesetzmässigkeiten. Ihr Anteil steigt, wenn das Phosphatierungsmittel im Unterschuss angewendet wird. Durch einen Überschuss an Phosphatierungsmittel ist die Bildung der Monoester begünstigt. Auch in diesem Falle gilt, dass Anteile an nicht umgesetzten, gegebenenfalls ethoxylierten Benzylalkanolen und aliphatischen GUERBET-Alkoholen bei den üblichen Anwendungen nicht stören.

Nach der Sulfatierung bzw. Phosphatierung werden die Teilester neutralisiert, vorzugsweise mit den Hydroxiden des Natriums, Kaliums, Ammoniums und Magnesiums. Gegebenenfalls können auch die Carbonate oder Hydrogencarbonate dieser Alkalimetalle verwendet werden. Als neutralisierende organische Basen kommen Monoethanolamin, Diethanolamin, Triethanolamin und die analogen Propanolamine sowie Guanidin in Frage. Die erhaltenen Salze sowie ihre Gemische mit den Salzen der Teilester der Formel IV

$$R^1-CH_2-CH_2-\underset{\underset{R^1}{|}}{CH}-CH_2O-(R^2O)_n-X \quad (IV)$$

die sich von nicht abgetrennten, aliphatischen GUERBET-Alkoholen ableiten, stellen wertvolle als Netzmittel, Dispergiermittel, Emulgiermittel und Waschrohstoffe dar. Hinsichtlich ihrer Reinigungskraft sind die Sulfate den analog aufgebauten Alkylphenolglykolethersulfaten und Alkylglykolethersulfaten überlegen. Sie sind biologisch gut abbaubar und nichttoxisch und unterscheiden sich hierin vorteilhaft von den analogen Alkylphenolderivaten.

*Beispiele*

*I. Darstellung der 2-Benzylalkanole*

A) 2-Benzyldodecanol-(1)

558,9 g (3 Mol) n-Dodecanol, 642,6 g (6 Mol) Benzylalkohol, 33,8 g (0,525 Mol) Kaliumhydroxid (eingesetzt als 87%ige wässrige Lösung) und 12 g feinteiliges Nickel (GIRDLER-Nickel) wurden unter Rühren auf 205 bis 210° C erhitzt. Abdestilierender Benzylalkohol wurde kondensiert, in einer Abscheidungsvorrichtung, die mit einer im Kreislauf geführten gesättigten Natriumsulfat-Lösung beschickt wurde, von Wasser befreit und in das Reaktionsgemisch zurückgeführt. Innerhalb 2 Stunden wurden 65 ml Wasser, in dem geringe Anteile Benzylalkohol gelöst waren, abgetrennt (theoretische Menge an Reaktionswasser 58,4 ml). Nach dem Abkühlen wurde der aus Katalysator und Kaliumbenzoat bestehende Rückstand abgetrennt und der überschüssige Benzylalkohol im Rotationsverdampfer abdestilliert.

Das erhaltene Produkt wurde über eine Fraktionierkolonne fraktionierend destilliert. Das bei einem Druck von 0,95 mb (Millibar) bei 178° C siedende 2-Benzyldodecanol-(1) wurde isoliert. Die Ausbeute betrug 632 g (74% der Theorie). Der Rückstand und ein Anteil von 3 Gew.-% im Destillat bestanden aus 2-Decyltetradecanol-(1).

Ein Teil des Destillats wurde nochmals fraktionierend destilliert und ein 2-Benzyldodecanol-(1) in einer Reinheit von 99,7% erhalten (Prüfung durch Gelchromatographie). Siedepunkt 133° C bei 0,136 mb (0,1 Torr); Brechungsindex $n_D^{20}$=1,4962 (Literaturwert $n_D^{20}$=1.4945).
Elementaranalyse: $C_{19}H_{32}O$ (276,5),
Hydroxylzahl: OHZ=201 (berechnet 202,8).

B) 2-Benzyltetradecanol-(1)

Die Umsetzung erfolgte wie unter (A) angegeben. Eingesetzt wurden 428,8 g (2 Mol) n-Tetradecanol-(1), 642,6 g (6 Mol) Benzylalkohol, 33,8 g (0,525 Mol) Kaliumhydroxid (87%ig) und 12 g Nickelkatalysator. Bei einer Temperatur von 205 bis 210° C wurden während 1,5 Stunden 45 ml Wasser abdestilliert. Nach dem Abtrennen des Katalysators, des Kaliumbenzoats und des überschüssigen Benzylalkohols wurden durch fraktionierende Destillation bei 198° C und 0,95 mb 420 g eines 92%igen 2-Benzyltetradecanols-(1) gewonnen (64% Ausbeute). Der Destillationsrückstand bestand im wesentlichen aus 2-Dodecylhexadecanol-(1). Durch erneute fraktionierte Destillation wurde das 2-Benzyltetradecanol in 98,1%iger Reinheit gewonnen. Siedepunkt 181° C bei 0,68 bar (0,05 Torr), $n_D^{20}$=1,4935; Elementaranalyse: $C_{21}H_{36}O$ (304,5), OHZ=182 (berechnet OHZ=184).

C) 2-Benzyloctanol-1

Wie vorstehend beschrieben, wurden 911,4 g (7 Mol) n-Octanol, 1513 g (14 Mol) Benzylalkohol, 79 g (1,225 Mol) Kaliumhydroxid (87%ig) und 24,3 g Nickel-Katalysator 6 Stunden auf Temperaturen zwischen 186 und 194° C erhitzt. Das entstehende Wasser (120 ml) wurde zusammen mit Octanol abdestilliert und unter Rückführung des Octanols abgeschieden. Nach dem Abkühlen und Abtrennen des Katalysators wurden nichtumgesetztes Octanol und überschüssiger Benzylalkohol abdestilliert und der Rückstand fraktioniert. Das bei 116° C/0,109 mb (0,08 Torr) in einer Menge von 1044 g erhaltene Destillat bestand zu 85 Gew.-% aus 2-Benzyloctanol-(1) und zu 15 Gew.-% aus 2-Hexyldecanol-(1). Wegen der sehr nahe beieinanderliegenden Siedepunkte der beiden Alkohole wurde auf eine weitere Fraktionierung verzichtet. Die OHZ des Gemisches betrug 240, der Brechungsindex $n_D^{20}$=1,4971.

D) 2-Benzyloctanol-(1) und 2-Benzyldecanol-(1)

Als Ausgangsmaterial diente ein technisches Gemisch aus n-Octanol und n-Decanol mit einem Molekulargewicht (MG) von 141,1. Von diesem Gemisch wurden 1128,8 g (8 Mol) zusammen mit 1729,6 g (16 Mol) Benzylalkohol, 90,3 g Kaliumhydroxid und 28,6 g Nickelkatalysator im Verlauf von 6 Stunden zum Sieden (195 bis 178° C) erhitzt und aus dem Destillat das Wasser abgeschieden. Die Aufarbeitung erfolgte wie unter D) beschrieben. Das erhaltene, bei 130° C/0,136 mb (0,1 Torr) siedende Alkoholgemisch, das noch 15 Gew.-% an aliphatischen GUERBET-Alkoholen aus Octanol und Decanol enthielt, wies die folgenden Kennzahlen auf: OHZ 222,5, $n_D^{20}$=1,4963.

*II. Darstellung der 2-Benzylalkanol-polyglykolether*

55,3 g (0,2 Mol) 2-Benzyldodecanol-(1) und 4,43 g Natriummethylat (100%ig, eingesetzt als 30%ige methanolische Lösung) wurden vermischt und nach Entfernen des Methanols im Vakuum in einem Autoklaven unter Rühren bei 160 bis 170° C im Verlauf von 30 Minuten mit 8,8 g (0,2 Mol) Ethylenoxid umgesetzt. Nach dem Abkühlen, dem Neutralisieren des Katalysators mit Essigsäure und Abtrennen des gebildeten Natriumacetats wurde ein schwach gelblich gefärbtes Produkt mit einer

OHZ von 180 erhalten. Der durchschnittliche Ethoxylierungsgrad beträgt 1.

Die in gleicher Weise hergestellten 2-Benzyldodecanol-polyglykolether sind in Tabelle 1 aufgeführt (n=Anzahl der EO-Gruppen).

In einer weiteren Versuchsreihe wurden, wie vorstehend beschrieben, 89,3 g (0,3 Mol), 97,8%iges 1-Benzyltetradecanol-(1) in Gegenwart von 7,8 g Natriummethylat nach Entfernen des Methanols im Vakuum mit 26,4 g (0,6 Mol) Ethylenoxid unter Druck bei 160 bis 170° C umgesetzt. Nach beendeter Umsetzung (Absinken des Drucks) wurde ein schwach gelblich gefärbtes, flüssiges Produkt erhalten. Die OHZ betrug 144.

*Tabelle 1*

| $R^1$ | n | Konsistenz 20° C | OHZ |
|---|---|---|---|
| 10 | 1 | flüssig | 180 |
| 10 | 2 | flüssig | 159 |
| 10 | 3 | flüssig | 137,5 |
| 12 | 2 | flüssig-pastös | 144 |

In gleicher Weise wurde das gemäss Versuch (C) erhaltene technische 2-Benzyloctanol-(1) mit einem Anteil von 15% an 2-Hexyldecanol-(1) sowie das gemäss Versuch (D) hergestellte Gemisch aus 2-Benzyloctanol-(1) und 2-Benzyldecanol-(1) mit einem Gehalt von ca. 15 Gew.-% an aliphatischen GUERBET-Alkoholen in Gegenwart von Natriummethylat ethoxyliert. Die erhaltenen Polyglykolether sind in Tabelle 2 zusammengestellt.

*Tabelle 2*

| $R^1$ | n | Konsistenz | OHZ |
|---|---|---|---|
| 6 | 2 | flüssig | 178 |
| 7/8 | 2 | flüssig | 168 |

*III. Darstellung der Teilester*

*Beispiel 1:*

70,6 g (0,2 Mol) 2-Benzyldodecanol-1-diglykolether (n=2) wurden unter Kühlung mit Eis und intensivem Rühren innerhalb von 15 Minuten mit 23,3 g (0,2 Mol) Chlorsulfonsäure versetzt. Die Temperatur des Reaktionsgemisches betrug 20 bis 25° C. Der entstehende Chlorwasserstoff wurde gleichzeitig bei einem Unterdruck von 267 mb (200 Torr) entfernt. Nach vollständiger Zugabe der Chlorsulfonsäure wurde zur Nachreaktion das Gemisch noch 30 Minuten im Temperaturbereich von 30 bis 35° C gerührt und der Druck zwecks Entfernung des restlichen Chlorwasserstoffs auf 20 mb (15 Torr) abgesenkt.

Der Schwefelsäurehalbester wurde unter Rühren in eine eisgekühlte Lösung von 8 g (0,2 Mol) Natriumhydroxid in 209 g Wasser gegeben. Es wurde eine fast farblose, transparente Paste mit folgenden analytischen Daten erhalten, wobei der Gehalt an Schwefelsäurehalbestersalz analytisch als $NaSO_3$-H bestimmt wird:

$NaSO_3$-H = 6,2% (theoretisch 6,7%)
Wasser = 70,0%
$SO_4$ = 0,2%
Cl = 0,1%
Sulfiergrad = 92%

*Beispiel 2:*

Wie in Beispiel 1 angegeben, wurden 81,6 g (0,2 Mol) 2-Benzyldodecanol-1-triglykolether (n=3) innerhalb 15 Minuten mit 24,1 g (0,2 Mol) Chlorsulfonsäure mittels Unterdruck entfernt. Die Nachreaktion erfolgte in der in Beispiel 1 angegebenen Weise.

Die Neutralisation erfolgte mit 8,2 g (0,205 Mol) Natriumhydroxid in 238 ml Wasser. Es wurde eine fast farblose, transparente Paste mit folgenden analytischen Daten erhalten:

$NaSO_3$-H = 5,7% (theoretisch 5,9%)
Wasser = 70,3%
$SO_4$ = 0,4%
Cl = 0,1%
Sulfiergrad = 96%

*Beispiele 3 bis 7:*

In gleicher Weise wurden die übrigen gemäss Kapitel II hergestellten 2-Benzylalkanol-1-polyglykolether sulfatiert und folgende Teilestersalze erhalten. Die Bezeichnung EO steht für die Glykolethergruppe.

3) 2-Benzyldodecanol-1-(EO)-$SO_3$Na
4) 2-Benzyltetradecanol-1-$(EO)_2$-$SO_3$Na
5) 2-Benzyloctanol-1-$(EO)_1$-$SO_2$Na
6) 2-Benzyloctanol-1-$(EO)_3$-$SO_3$Na
7) Gemisch aus
   2-Benzyloctanol-1-$(EO)_2$-$SO_3$Na
   2-Benzyldecanol-1-$(EO)_2$-$SO_3$Na

Das Gemisch gemäss Beispiel 7 enthielt zusätzlich noch ca. 15% sulfatierte aliphatische GUERBET-alkohol-ethoxylate.

*IV. Anwendungstechnische Prüfung*

Als Testsubstanzen dienten Verbindungen gemäss Beispiel 1 und 2 sowie 5 bis 7. Als Vergleichssubstanzen wurden herangezogen:

V1) Alkyl-$(EO)_2$-$SO_3$Na (Cocos-Alkyl)
V2) n-Dodecylbenzolsulfonat–Na.

a) *Schaummessungen*

Die Prüfung des Schaumvermögens wurde nach der «Lochscheiben-Schlagmethode» gemäss DIN 53902 (Blatt 2) in Wasser von 16 dH (160 mg CaO pro Liter) bei 30° C durchgeführt. Es wurden Gemische aus 1 Gewichtsteil des jeweiligen Tensids und 3 Gewichtsteilen Natriumsulfat in steigender Konzentration eingesetzt. Die Schaumhöhe (in mm) ist der Tabelle 3 zu entnehmen.

*(Tabelle auf den nächsten Seiten)*

Die Dodecanol- und Tetradecanol-Derivate zeigen eine vergleichsweise geringe Schaumentwicklung und eignen sich daher besonders für schaumschwache, in Waschmaschinen einsetzbare Zubereitungen. Die kürzerkettigen Derivate entsprechen in ihrem Schaumverhalten den Vergleichsprodukten.

Tabelle 3

| Tensid gemäss Beispiel | Schaumhöhe in mm bei Einsatz von | | | | |
|---|---|---|---|---|---|
| | 1 g/l | 1,5 g/l | 2 g/l | 3 g/l | 4 g/l |
| 1 | 50 | 100 | 110 | 180 | 220 |
| 2 | 90 | 110 | 120 | 210 | 230 |
| 5 | 270 | 320 | 400 | 500 | 580 |
| 6 | 270 | 340 | 420 | 500 | 600 |
| 7 | 220 | 300 | 360 | 440 | 510 |
| V1 | 310 | 400 | 550 | 590 | 620 |
| V2 | 290 | 350 | 420 | 480 | 600 |

b) *Feinwaschvermögen*

Zur Prüfung des Wollwaschvermögens wurden Stränge aus Wollgarn, die unter standardisierten Bedingungen angeschmutzt worden waren, in einer Garnwaschmaschine bei 30° C gewaschen. Es wurden die gleichen Gemische aus Tensid und Natriumsulfat eingesetzt wie im Schaumtest (Versuchsreihe a). Das Flottenverhältnis (Garngewicht zu Liter Waschlösung) betrug 1:29 und die Wasserhärte 16° dH. Nach dem Waschen (Waschzeit 15 Minuten) wurden die Proben 3 mal mit Leitungswasser gespült und getrocknet. Die Ergebnisse der photometrischen Remissionsmessungen sind in Tabelle 4 zusammengestellt.

Tabelle 4

| Tensid gemäss Beispiel | Remission in Abhängigkeit von der Konzentration | | | | |
|---|---|---|---|---|---|
| | 1 g/l | 1,5 g/l | 2 g/l | 3 g/l | 4 g/l |
| 1 | 39,7 | 46,0 | 54,8 | 57,9 | 61,0 |
| 2 | 30,3 | 44,1 | 46,8 | 53,5 | 56,9 |
| V1 | 28,4 | 31,4 | 42,6 | 46,0 | 50,3 |
| V2 | 20,3 | 21,5 | 32,2 | 39,2 | 51,2 |

In einer zweiten Versuchsreihe wurden 0,5 g/l Tensid und 1,5 g/l Natriumsulfat eingesetzt und Wasser mit unterschiedlichem Härtegrad verwendet. Die Ergebnisse sind in Tabelle 5 aufgeführt.

Tabelle 5

| Tensid gemäss Beispiel | Remission in Abhängigkeit von der Härte | | | | |
|---|---|---|---|---|---|
| | 0° dH | 4° dH | 8° dH | 16° dH | 32° H |
| 1 | 48,1 | 54,4 | 54,2 | 49,5 | 48,7 |
| 2 | 43,3 | 47,7 | 48,2 | 46,3 | 43,4 |
| V1 | 39,3 | 39,5 | 41,6 | 42,3 | 39,4 |
| V2 | 38,7 | 36,4 | 30,7 | 29,3 | 23,4 |

Die erfindungsgemässen Tenside zeichnen sich durch überlegene Wascheigenschaften und Unempfindlichkeit gegen hartes Wasser aus.

c) *Waschvermögen bei mittleren bis höheren Temperaturen*

Die folgenden Versuche wurden im Launderometer durchgeführt, wobei unter standardisierten Bedingungen mit Hautfett und mineralischem Schmutz angeschmutzte Textilproben aus Baumwolle (B), veredelter Baumwolle (Bv), Mischgewebe aus veredelter Baumwolle und Polyesterfaser (BvP) sowie aus Polyesterfaser (P) gewaschen wurden. Als Waschmittel wurden 0,75 g/l Tensid, 1 g/l Natriumtripolyphosphat und 1 g/l eines feinkristallinen, synthetischen Zeoliths vom Typ NaA verwendet. Die Wasserhärte betrug 16° dH und die Waschtemperatur 90° C bei Baumwolle bzw. 60° C bei den übrigen Geweben. Das Flottenverhältnis betrug 1:12 bei Baumwolle und 1:30 bei den übrigen Geweben. Die Waschtrommel wurde jeweils mit 4,2 g Testgewebe und 4,2 g sauberem Füllgewebe (Baumwolle) sowie 10 Stahlkugeln gefüllt. Die Waschzeit betrug jeweils 15 Minuten. Anschliessend wurde 3 mal mit Leitungswasser nachgespült (16° dH). Die Auswertung erfolgte photometrisch. Die Ergebnisse sind in Tabelle 6 zusammengestellt.

Tabelle 6

| Textilmaterial:<br>Waschtemperatur: | B<br>95° | Bv<br>60° | BvP<br>60° | P<br>60° |
|---|---|---|---|---|
| Tensid gemäss Beispiel | % Remission | | | |
| 1 | 49,4 | 27,0 | 27,2 | 28, 2 |
| 2 | 42,8 | 36,0 | 25,7 | 26,4 |
| V2 | 35,1 | 23,8 | 23,2 | 22,0 |

d) *Benetzungseigenschaften*

Die Netzmittelwirkung wurde mittels Tauchnetztest unter Einsatz von 1 g/l Tensid bei 20° C in Wasser von 0° dH durchgeführt. In Tabelle 7 ist die Zeit in Sekunden angegeben, nach der das in die Lösung eingetauchte Testgewebe aus Baumwolle sich von der Haltevorrichtung löst und untersinkt.

Tabelle 7

| Tensid nach Beispiel | Tauchzeit sec. |
|---|---|
| 1 | 35 |
| 2 | 36 |
| 5 | 24 |
| 6 | 27 |
| V1 | 43 |

Die Mittel zeigen gegenüber der Vergleichssubstanz eine verbesserte Netzmittelwirkung.

*V. Biologische Abbaufähigkeit*

Nach dem OECD-Screening-Test erfüllen die nach den Beispielen 1 bis 7 hergestellten Verbindungen die Anforderungen der RVO des Waschmittelgesetzes. Nach 19 Tagen betrug die BiAS-Abnahme 99%.

## Patentansprüche

1. Teilester von 2-Benzylalkanolen-(1) und 2-Benzylalkanol-1-polyglykolethern der allgemeinen Formel I

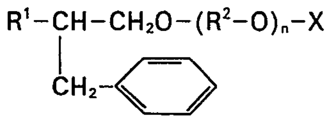
(I)

in der $R^1$ einen Alkylrest mit 6 bis 22 C-Atomen, $R^2$ einen Alkylenrest mit 2 bis 4 C-Atomen, n ganze Zahlen von 0 bis 10, X den Rest $SO_3Y$ oder $PO_3Y_2$ und Y Wasserstoff, ein Alkalimetall. Erdalkalimetall, Ammonium oder eine organische Ammoniumverbindung bedeuten.

2. Verfahren zur Herstellung der Verbindungen der Formel I, dadurch gekennzeichnet, dass man 1 Mol eines Alkanols, enthaltend 8 bis 24 C-Atome, zusammen mit 1 bis 10 Mol, vorzugsweise 2 bis 3 Mol Benzylalkohol in Gegenwart einer alkalisch reagierenden Alkalimetallverbindung und gegebenenfalls eines Hydrierungskatalysators auf Temperaturen von 180 bis 250° C erhitzt, das erhaltene 2-Benzylalkanol-(1) sowie dessen Gemisch mit Alkoholen der Formel II

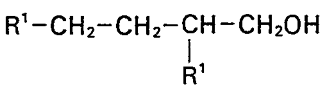
(II)

nach Abtrennung der übrigen Reaktionspartner entweder unmittelbar oder nach Überführung in die Alkylenoxid-Derivate in an sich bekannter Weise in die Schwefelsäureteilester oder Phosphorsäureteilester überführt und diese neutralisiert.

3. Verwendung der Verbindungen der Formel I und ihrer Gemische mit Teilestern von ethoxylierten Alkoholen der Formel IV

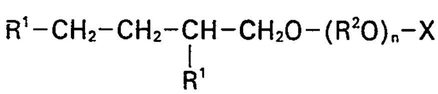
(IV)

als Netz-, Dispergier-, Emulgier- und Reinigungsmittel.

## Claims

1. Partial esters of 2-benzyl-1-alkanols and 2-benzyl-1-alkanol polyglycol ethers corresponding to general formula I

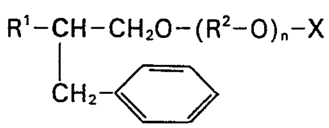
(I)

in which
$R^1$ is a $C_6$-$C_{22}$ alkyl radical, $R^2$ is a $C_2$-$C_4$ alkylene radical,
n is an integer of 0 to 10, X is the group $SO_3Y$ or $PO_3Y_2$ and
Y is hydrogen, an alkali metal, an alkaline earth metal, ammonium or an organic ammonium compound.

2. A process for the production of the compounds claimed in claim 1, characterized in that 1 mol of an alkanol containing 8 to 24 C atoms and 1 to 10 mol and preferably 2 to 3 mol benzyl alcohol are heated to temperatures of from 180 to 250° C in the presence of an alkaline-reacting alkali metal compound and, optionally, a hydrogenation catalyst, the 2-benzyl-1-alkanol obtained and its mixture with alcohols corresponding to formula II

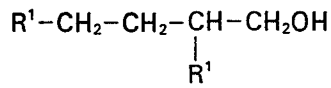
(II)

after separation of the other reactants is converted in known manner into the sulfuric acid partial ester or phosphoric acid partial ester either directly or after conversion into the alkylene oxide derivatives and the partial ester is neutralized.

3. The use of the compounds corresponding to formula I and mixtures thereof with partial esters of ethoxylated alcohols corresponding to formula IV

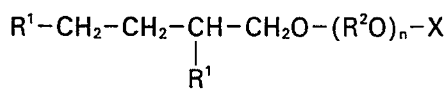
(IV)

as wetting agents, dispersants, emulsifiers and cleaning agents.

## Revendications

1. Ester partiel de 2-benzylalcanols (1) et d'éther de 2-benzylalcanol-1 et polyglycol de formule générale I

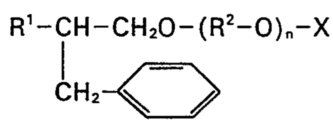
(I)

dans laquelle $R_1$ représente un reste alcoyle ayant de 6 à 22 atomes de C, $R_2$ représente un reste alcoylène ayant de 2 à 4 atomes de C, n un nombre entier de 0 à 10, X le reste $SO_3Y$ ou $PO_3Y_2$ et Y l'hydrogène, un métal alcalin, un métal alcalino-terreux, de l'ammonium, ou un sel d'ammonium organique.

2. Procédé d'obtention des composés de formule I, caractérisé en ce que l'on chauffe un mole d'un alcanol ayant de 8 à 24 atomes de carbone ensemble avec 1 à 10 moles, de préférence 2 à 3 moles d'alcool benzylique en présence d'un composé de métal alcalin à réaction alcaline et le cas échéant un catalyseur d'hydrogénation à des températures allant de 180 à 250°, le 2-benzylalcanol

(1) obtenu ainsi que son mélange avec des alcools de formule II

$$R^1-CH_2-CH_2-\underset{\displaystyle R^1}{\underset{|}{CH}}-CH_2OH \qquad (II)$$

après séparation des partenaires de la réaction usuels, soit immédiatement, soit après transformation en dérivé oxyde d'alcoylène d'une manière connue en soi, est transformé en l'ester partiel sulfurique ou l'ester partiel phosphorique et celui-ci est neutralisé.

3. Utilisation des composés de formule I et de leurs mélanges avec des esters partiels d'alcools éthoxylés de formule IV

$$R^1-CH_2-CH_2-\underset{\displaystyle R^1}{\underset{|}{CH}}-CH_2O-(R^2O)_n-X \qquad (IV)$$

comme agents de réticulation, dispersants, émulsifiants ou de nettoyage.